# EUROPEAN PATENT SPECIFICATION

(11) **EP 2 218 441 B1**
(45) Date of publication and mention of the grant of the patent: **25.05.2011**
(21) Application number: 09152983.4
(22) Date of filing: 17.02.2009
(51) Int. Cl.: A61K 8/60, A61K 8/67, A61Q 19/02

(54) **Methods for the prevention and/or treatment of age spots**
Verfahren zur Verhinderung und/oder Behandlung von Altersflecken
Procédés de prévention et/ou traitement de tâches de vieillesse

(43) Date of publication of application: 18.08.2010
(73) Proprietor: Rahn Ag, 8050 Zürich (CH)
(72) Inventor: Ebauer, Margret., 8048 Zürich (CH); Ott, Roman., 8315 Lindau (CH); Walzel, Bernd., 8046 Zürich (CH)
(74) Representative: Schnabel, Jörg

(56) References cited:
- WO-A-2008/006581
- DE-A1-102007 022 448
- US-A1- 2003 133 958
- US-A1- 2003 152 656

## Description

The present invention relates to a cosmetic method using oleuropein, optionally in combination with ascorbyl phosphate, for the prevention and/or treatment of age spots.

One of the phenomena of aging skin is the appearance of so-called age spots (Lentigo senilis). Age spots develop from strongly pigmented basal cells which grow in the direction of the dermis into so-called "buds" and form a network such that these cells do not migrate upwards as the normal keratinocytes do. It is believed that the accumulation of melanin and lipofuscin are the cause of the pigmentation.

The biochemical background of the appearance of age spots ultimately lies in the accumulation of oxidized proteins over the time. Normally, such oxidized proteins are degraded by the proteasome and exported from the cells. However, the activity of the proteasome diminishes over time. As a consequence, more and more oxidatively degraded proteins are accumulated, and an aggregate of oxidized proteins and lipids, so-called lipofuscin is formed. In the aggregated form of lipofuscin, the degraded proteins can not be processed by the proteasome anymore. The accumulation of lipofuscin in the cytoplasm is toxic for the cell. Thus, the more lipofuscin is present in a cell, the shorter is the cell's survival time.

WO-A-2008/006581 describes compositions containing hydroxytyrosol and/or oleuropein and at least one additional component which may be ascorbyl phosphate. The compositions according to WO-A-2008/006581 are said to be of use against inflammatory disorders, and, in this respect, the composition shall be used against photoageing.

Obviously, there is a need for a cosmetic regimen against age spots taking account of the biochemical and physiological, respectively, processes leading to their appearance.

The solution to this technical problem is provided by the embodiments of the present invention characterized in the claims.

In particular, the present invention provides the use of oleuropein for the prevention and/or treatment of age spots.

The present inventors have surprisingly found that oleuropein reduces the production of lipofuscin when keratinocytes are put under oxidative stress, which may be caused by an activation of the proteasome.

Therefore, the present invention provides a cosmetic method for the prevention and/or treatment of age spots comprising the step of topically applying an effective amount of oleuropein to human skin.

According to the present invention the treatment of age spots leads at least to their brightening.

In the method of the present invention, oleuropein is preferably applied in the form of a cosmetic or dermatological, respectively, composition containing oleuropein and at least one cosmetically acceptable carrier.

"Oleuropein" according to the present invention may originate from synthetic sources, e.g. it may be provided by chemical synthesis or from natural sources, e.g. extraction form Olive leafs. In particular if derived from natural sources, oleuropein is often present modified, e.g. hydroxylated or glycosylated. In the context of the present invention the term "oleuropein" therefore embraces such modified, in particular hydroxylated and/or glycosylated, forms as well.

According to preferred embodiments of the present invention, the cosmetic method comprises applying (an effective amount of) ascorbyl phosphate to the human skin, in addition to oleuropein.

The combination of oleuropein and ascorbyl phosphate provides a synergistic treatment of age spots, since oleuropein reduces the development of lipofuscin and ascorbyl phosphates effectively bleaches the age spots.

The term "ascorbyl phosphate" as used herein denotes metal salts of mono- and poly-phosphoric acid esters of ascorbic acid wherein the phosphorylated hydroxy group of the ascorbic acid molecule features one or more phosphoric acid (phosphate) units, and metal cations, e g sodium and/or magnesium or calcium ions, are also present. The term "poly" generally denotes 2-10, preferably 2-4. phosphate units. The ascorbyl phosphates may also be referred to in general as "ascorbyl (poly)phosphates" to embrace both mono- and polyphosphates. Typical ascorbyl phosphates for use in the present invention are L-ascorbic acid phosphate ester salts such as sodium ascorbyl phosphate, potassium ascorbyl phosphate, magnesium ascorbyl phosphate, calcium ascorbyl phosphate and sodium magnesium L-ascorbyl-2-monophosphate.

In the cosmetic method of the present invention, oleuropein and ascorbyl phosphate may be applied sequentially or simultaneously to the human skin.

Preferably, both active ingredients, oleuropein and ascorbyl phosphate are present either in the form of two distinct compositions, when oleuropein and ascorbyl phosphate are applied sequentially to the human skin, or they are present together in a composition in combination with at least one cosmetically acceptable carrier.

The term "effective amount" as used herein means an amount of the active ingredient, in particular oleuropein, optionally together with ascorbyl phosphate, leading to the desired effect. Preferably, in case of corresponding cosmetical or dermatological compositions, the effective amount is at least 0.001 %, more preferred 0.01 to 20 %, even more preferred 0.05 and 10 %, still more preferred 0.1 and 5 %, of each of the active agents of the present invention, based on the total weight of the cosmetic composition.

"Cosmetically acceptable carrier" relates to a corresponding base composition comprising conventional cosmetic adjuvants and/or additives and/or additional active ingredients.

Thus, the cosmetic preparations of the present invention are preferably skin care formulations for the treatment and/or prevention of age spots.

The amount of the cosmetic preparation/composition which is to be applied to the skin depends on the concentration of the active ingredient(s), i. e. oleuropein, optionally together with ascorbyl phosphate, in the composition and on the desired cosmetic effect. For example, the application of the cosmetic/dermatological composition can be such that a cream is applied to the skin.

A cream is usually applied in amount of about 1 to 2 mgr cream/cm² skin. The amount of the composition applied to the skin is, however, not critical, and if, with a certain amount of applied composition, the desired effect, i. e. prevention/brightening or bleaching of age spots, can not be achieved, a higher concentration of the active preparations containing more active ingredients might be employed.

The cosmetic preparations/compositions of use in the present invention may be in the form of a suspension or dispersion in solvents or fatty substances, or altenatively in the form of an emulsion or micro emulsion (in particular, of O/W or W/O type, O/W/O or W/O/W-type, wherein O stands for oil phase and W stands for water phase), such as a cream, a paste, a lotion, a thickened oil or a milk, a vesicular dispersion in the form of an ointment, a gel, a solid tube stick or an aerosol mousse, may be provided in the form of a mousse, for a foam or spray foams, sprays, sticks or aerosols or vibes. Examples of cosmetic or dermatological preparations are skin care preparations, in particular, body oils, body lotions, body gels, treatment creams, skin protection ointments, moisturizing gels, moisturizing sprays, revitalizing body sprays, after-sun preparations or sun cream formulations.

Cosmetic compositions for use in the present invention may further comprise usual cosmetic adjuvants and/or additives such as preservatives/antioxidants, fatty substances/oils, water, organic solvents, silicones, thickeners, softeners, emulsifiers, additional light screening agents, antifoaming agents, moisturizers, frequenters, surfactants, fillers, sequestering agents, anionic, cationic, non-ionic or amphoteric polymers or mixtures thereof, propellants, acidifying or basifying agents, dyes, colorants, pigments or nanopigments, light stabilizers, insect propellants, skin tanning agents, skin whitening agents, antibacterial agents, preservative active ingredients or any other ingredients usually formulated into cosmetic or dermatological preparations.

The necessary amount of the cosmetic adjuvants, additives and/or additional active ingredients can, based on the desired product, easily be chosen by a person skilled in the art.

As an example of a preferred cosmetic composition of the invention, said composition may contain
about 0.001 to 8 weight %, preferably about 0.02 to 0.5 weight %, more preferably about 0.05 to 0.3 weight % of oleuropein;
about 0.01 to 8 weight %, preferably 0.5 to 5 weight%, more preferably about 1 to 3 weight %, most preferred about 2 weight % of ascorbyl phosphate;
about 84.00 to 99.989 weight % of the cosmetically acceptable carrier as defined above.

The figures show:

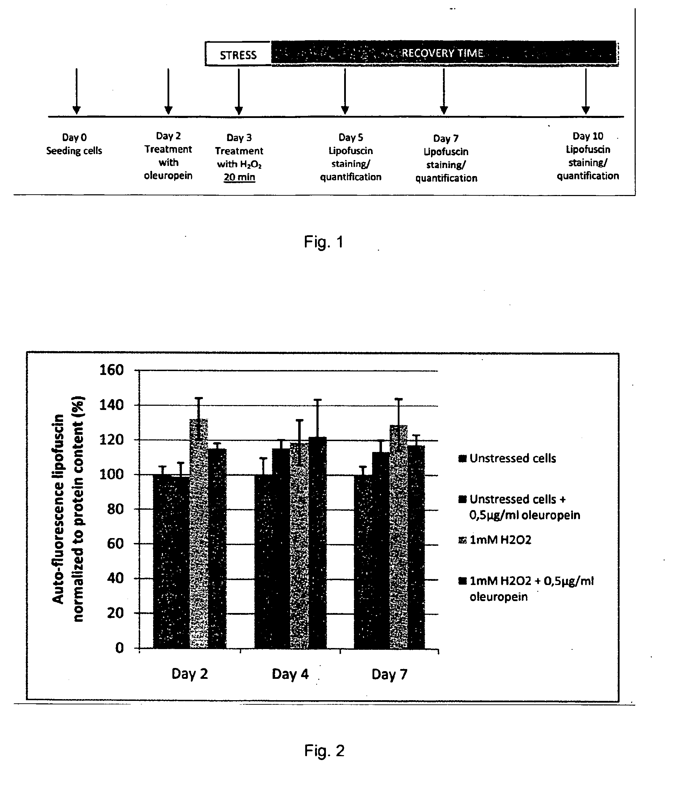

- Figure 1:: shows a schematic overview of a protocol for testing the efficacy of oleuropein in preventing the production of lipofuscin by human keratinocytes upon inducing oxidative stress using H₂O₂.
- Figure 2:: shows a graphic representation of the results of lipofuscin autofluorescence by human keratinocytes stressed with 1 mM H₂O₂ for 20 minutes and treated with 0.5 µg/ml oleuropein or untreated. Lipofuscin autofluorescence was measured 2, 4 and 7 days after oxidative stress and normalized to the total protein content. The values shown are means ± standard deviation for 3 independent experiments.

The following non-limiting examples further illustrate the present invention.

### EXAMPLES

### Example 1

### Oleuropein reduces lipofuscin formation in keratinocytes under oxidative stress

Accumulating with age, lipofuscin is one of the highlights of the mechanism underlying formation of age spots. Lipofuscin is a highly oxidized-linked aggregate consisting of oxidized protein and lipid. These cross-linked proteins are insoluble, not degradable by lysosomal enzymes or proteasomal system and are mainly present into the cytosol compartment (Jung et al, 2007).

Because there is no specific lipofuscin antibody and its composition varies between different cell types, lipofuscin autofluorescence is the most widely used method for its detection and quantification. In order to evaluate the effect of oleuropein on lipofuscin formation in oxidatively stressed cells, lipofuscin accumulation was measured by autofluorescence, after induction of oxidative stress to human keratinocytes, treated or not with oleuropein.

### Experimental overview

As shown in Fig. 1, cultured human keratinocytes were maintained 2 days in culture before they were treated with 0.5µg/ml oleuropein. After 24h incubation with oleuropein, the cells were treated with 1 mM H₂O₂ for 20 minutes. After a recovery time of 2, 4 or 7 days, representative pictures of cells were taken and lipofuscin amount was quantified by autofluorescence measurement.

### Materials and methods

### Cell culture

Cells were human keratinocytes (NHKs) cultured in Epilife medium (Cascade Biologics, Portland, USA) containing HKGS (Cascade Biologics, Portland, USA) and supplemented with gentamycin (Invitrogen, UK). NHKs were maintained in a humidified incubator at 37°C with a 5% CO₂ atmosphere, kept in culture under exponential growth conditions and harvested with trypsin/ethylenediaminetetraacetate solution.

### Oleuropein

Oleuropein (#89338, Phytolab, Germany) was stored in the dark at 4°C until needed.

### Oxidative stress

Cells present in 6-well plates were put under oxidative stress as follows: different densities of cells (30.000, 50.000, 75.000 and 100.000 cells per well) were seeded to define the best culture conditions to induce oxidative stress following the scheme illustrated in Fig. 1. The density of 30 000 cells per well was shown to be the best condition to see an accumulation of lipofuscin after oxidative stress.
Before treatment, cells were incubated during 2 days at 37°C and 5% CO₂. Thereafter, cells were treated or not with 0.5µg/ml oleuropein. The oxidative stress was performed at day 3, 24 hours after the treatment with oleuropein. The oxidative stress treatement consists on an incubation of cells with 1 mM H₂O₂. A 20 minutes exposure time was shown to be the most efficient to induce cell premature senescence and lipofuscin accumulation, without affecting viability. After oxidative stress, cells were incubated at 37°C and 5%CO₂, in the presence or absence of oleuropein, during a recovery time of 2, 4 or 7 days.

### Lipofuscin autofluorescence detection

After 2, 4 and 7 days, respectively, representative pictures of the cells were taken, and lipofuscin autofluorescence was measured.

The total protein content was determined at each time-point for data normalization purposes. Pictures of cells were taken using a Leica DM100 microscope (lens 20X and 40X) and DCF290 camera.

The autofluorescence of lipofuscin was detected at wavenlengths of 570-605nm after an excitation at 366nm, using a fluorometer (Vistor2TM, PerkinElmer, Belgium).

For each condition tested, the quantity of autofluorescent lipofuscin was normalized to the amount of total proteins Proteins were extracted with M-PER lysis buffer (Mammalian Protein Extraction Reagent, Pierce, USA), according the manufacturer's instructions. The dosage of proteins was performed following the Bradford method, based on the detection of the absorbance of Coomassie Blue (Coomassie Protein Assay Kit, Pierce, USA). The optical density was measured at 595nm using a microplate reader.

### Results

The results of the lipofuscin autofluorescence measurements are graphically depicted in Fig. 2,wherein the lipofuscin content is expressed as the amount of lipofuscin related to total protein contents, in percentage of the control.

Both at days 2, 4 and 7, H₂O₂ induced an increase of lipofuscin accumulation (increase of 32, 18 and 29% as compared to control cells without oxidative stress). The most significant effect was observed 2 days after the oxidative stress. At that time, 0,5 µg/ml oleuropein significantly decreased lipofuscin formation, as compared to untreated H₂O₂- exposed cells (decrease of 13%).

### Conclusions

Oxidized and cross-linked proteins (lipofuscin, age pigments, ceroid, etc.) have long been known to accumulate in aging and in age-related diseases, and some studies have suggested that age-dependent inhibition of the proteasome and/or lysosomal proteases may contribute to this phenomenon (Grune et al., 2005).

The intracellular formation of lipofuscin is a complex network of reactions involving various cellular compartment and enzymes, including lysosomes, mitochondria, the proteasomal system, the cytosol, and perhaps, the already existing lipofuscin. While lipofuscin is demonstrated not degradable by lysosomal enzymes or proteasomal system (Jung et al, 2007), the proteasome is inhibited by lipofuscin, perhaps as a result of binding proteasomal complex in unsuccessful attempts of degradation. Therefore, lipofuscin is directly able to decrease cellular proteolysis by inhibition of the proteasomal turnover, resulting in reduced proteasomal activity (Sitte et al., 2000, Friguet et al., 1997).

In the present experiments, the effect of oleuropein on the accumulation of lipofuscin triggered by an oxidative stress was evaluated and it could be demonstrated that 2 days after the stress, there was a reduction of lipofuscin by 13% when human keratinocytes were exposed to 1 mM H₂O₂ for 20 minutes and treated with oleuropein.

### Summary

To assess the effect of oleuropein in preventing the formation of lipofuscin in human keratinocytes, the formation of lipofuscin aggregates was induced by submitting cells to subcytotoxic oxidative stress with 1 mM H₂O₂, for 20 minutes. Thereafter, the content of lipofuscin into stressed cells treated or not with 0.5µg/ml oleuropein, by autofluorescence detection was compared. After a recovery time of 2 days, it was demonstrated that the amount of lipofuscin following H₂O₂ exposure was increased by 32% as compared to control cells. When cells were pre-treated with 0.5µg/ml oleuropein for 24 hours before and 2 days after the stress, the content of lipofuscin was reduced by 13% as compared to stressed cells not treated with oleuropein.

### References

Jung et al., Formation, distribution, and metabolic consequences, Ann. N.Y. Acad. Sci 1119: 97-111, 2007.
Grune et al., Protein oxidation and degradation during postmitotic senescence, Free Radic Biol Med., Nov 1;39(9):1208-15, 2005.
Sitte et al., Proteasome inhibition by lipofuscin/ceroid during posmitotic aging of fibroblasts, FASEB J,14, 1490-1498, 2000.
Friguet et al., Inhibition of the multicatalytic proteinase (proteasome) by 4-hydroxy-2-nonenal crosslinked protein, FEBS Letters, Volume 405 (1), 21 - 25, 1997.
Bruke and Skumatz, Autofluorescent inclusions in long-term postconfluent cultures retinal pigment epithelium, Invest Ophtalmol Vis Sci, 39: 1478-1486, 1998.

### Example 2

The following is a preferred O/W body cream according to the present invention:

### Components

| **O/W Body Cream** | | |
|---|---|---|
| | pH-value appearance | approx. 6.5 Oil in water emulsion |
| **Phase** | **Substance** | **% [w/w]** |
| A | Water | 65.70 |
| | Glycerin | 3.00 |
| B | Glyceryl Stearate Citrate | 3.00 |
| | Sucrose Stearate | 1.00 |
| | Cetearyl Alcohol | 3.00 |
| | Ethylhexyl Palmitate | 9.00 |
| | Cetearyl Isononanoate | 8.00 |
| | Phenoxyethanol, | 1.00 |
| | Ethylhexylglycerin | |
| | Dimethicone | 3.00 |
| C | Xanthan Gum | 0.25 |
| | Carbomer | 0.15 |
| D | Sodium Hydroxide, Aqua | 0.60 |
| E | Oleuropein | 0.25 |
| F | Ascorbyl Phosphate | 2.00 |
| G | Fragrance | 0.05 |

### Method of preparation

### Prepare phase A, heat to 75°C;

mix components of phase B, stir until the oils moisten the surfactant, then heat to 75°C;
mix components of phase C, add it to the hot phase B, stir shortly;
add the mixture of phases B/C to phase A, homogenize shortly;
add component D, homogenise strongly, cool to 40°C whilst stirring;
add components E, F and separately, cool to 25°C whilst stirring.

### Example 3

The following is a preferred W/O cream according to the present invention:

### Components

| **W/O Cream** | | |
|---|---|---|
| | appearance specialities | white emulsion water-in-oil emulsion |
| **Phase** | **Substance** | **% [w/w]** |
| A | Water | 69.20 |
| | Magnesium Sulfate | 0.70 |
| | Glycerin | 5.00 |
| B | Sorbitan Oleate, | 5.00 |
| | Polyglyceryl-3 Polyricinoleate | |
| | Ethylhexyl Palmitate | 6.00 |
| | Decyl Oleate | 5.00 |
| | Dicaprylyl Ether | 2.00 |
| | Phenoxyethanol, | 1.00 |
| | Ethylhexylglycerin | |
| | Cera Alba | 3.00 |
| | Sucrose Polystearate | 1.00 |
| C | Oleuropein | 0.05 |
| D | Ascorbyl Phosphate | 1.95 |
| E | Fragrance | 0.10 |

### Method of preparation

Mix components of phase A, heat to 85°C;
mix components of phase B, heat to 85°C whilst stirring;
add phase A very slowly to phase B always under stirring; homogenise strongly;
cool down to 40°C whilst stirring;
add components C, D and E, homogenize again;
cool down to 25°C whilst stirring.

## Claims

1. Cosmetic method for the prevention and/or treatment of age spots comprising the step of topically applying an effective amount of oleuropein to human skin.

2. The method of claim 1 wherein the oleuropein is applied in the form of a cosmetic composition containing oleuropein and at least one cosmetically acceptable carrier.

3. The method of claim 1 or 2 further comprising applying ascorbyl phosphate to the human skin.

4. The method of claim 3 wherein oleuropein and ascorbyl phosphate are applied sequentially or simultaneously to the human skin.

5. The method of claim 4 wherein oleuropein and ascorbyl phosphate are present in a composition containing oleuropein and ascorbyl phosphate together with at least one cosmetically acceptable carrier.

6. The method of claim 5 wherein the composition contains
0.001 to 8 weight % of oleuropein;
0.01 to 8 weight % of ascorbyl phosphate;
84.00 to 99.989 weight % of the cosmetically acceptable carrier.

7. The method according to any one of the preceding claims wherein the ascorbyl phosphate is sodium ascorbyl phosphate or potassium ascorbyl phosphate.

8. Cosmetic use of oleuropein for the prevention and/or treatment of age spots.

9. The use of claim 8 wherein oleuropein is used together with ascorbyl phosphate.

## Patentansprüche

1. Kosmetisches Verfahren zur Vorbeugung und/oder Behandlung von Altersflecken, umfassend das topische Auftragen einer wirksamen Menge Oleuropein auf die menschliche Haut.

2. Verfahren nach Anspruch 1, wobei Oleuropein in Form einer kosmetischen Zusammensetzung aufgetragen wird, welche Oleuropein und mindestens einen kosmetisch verträglichen Träger enthält.

3. Verfahren nach Ansprüchen 1 oder 2, weiterhin umfassend das Auftragen von Ascorbylphosphat auf die menschliche Haut.

4. Verfahren nach Anspruch 3, wobei Oleuropein und Ascorbylphosphat sequenziell oder gleichzeitig auf die Haut aufgetragen werden.

5. Verfahren nach Anspruch 4, bei dem Oleuropein und Ascorbylphosphat in einer Zusammensetzung vorliegen, die Oleuropein und Ascorbylphosphat zusammen mit mindestens einem kosmetisch verträglichen Träger enthält.

6. Verfahren nach Anspruch 5, wobei die Zusammensetzung
0,001 bis 8 Gew.-% Oleuropein,
0,01 bis 8 Gew.-% Ascorbylphosphat,
84,00 bis 99,989 Gew.-% kosmetisch verträglichen Träger enthält.

7. Verfahren nach einem der vorhergehenden Ansprüche, wobei das Ascorbylphosphat Natrium-Ascorbylphosphat oder Kalium-Ascorbylphosphat ist.

8. Kosmetische Verwendung von Oleuropein zur Vorbeugung und/oder Behandlung von Altersflecken.

9. Verwendung von Anspruch 8, wobei Oleuropein zusammen mit Ascorbylphosphat verwendet wird.

## Revendications

1. Procédé cosmétique de prévention et/ou traitement de tâches de vieillesse comprenant l'étape d'application topique d'une quantité efficace d'oleuropéine sur la peau humaine.

2. Procédé selon la revendication 1, dans lequel l'oleuropéine est appliquée sous la forme d'une composition cosmétique contenant de l'oleuropéine et au moins un support acceptable en cosmétique.

3. Procédé selon la revendication 1 ou 2, comprenant en outre l'application de phosphate d'ascorbyle sur la peau humaine.

4. Procédé selon la revendication 3, dans lequel l'oleuropéine et le phosphate d'ascorbyle sont appliqués séquentiellement ou simultanément sur la peau humaine.

5. Procédé selon la revendication 4, dans lequel l'oleuropéine et le phosphate d'ascorbyle sont présents dans une composition contenant de l'oleuropéine et du phosphate d'ascorbyle avec au moins un support acceptable en cosmétique.

6. Procédé selon la revendication 5, dans lequel la composition contient
0.001 à 8% en poids d'oleuropéine ;
0.01 à 8% en poids de phosphate d'ascorbyle ;
84.00 à 99.989% en poids de support acceptable en cosmétique.

7. Procédé selon l'une quelconque des revendications précédentes, dans lequel le phosphate d'ascorbyle est du phosphate d'ascorbyle de sodium ou du phosphate d'ascorbyle de potassium.

8. Utilisation cosmétique d'oleuropéine, pour la prévention ou le traitement de tâches de vieillesse.

9. Utilisation selon la revendication 8, dans laquelle l'oleuropéine est utilisée avec du phosphate d'ascorbyle.
